# EUROPÄISCHE PATENTSCHRIFT

(11) **EP 4 599 861 B1**
(45) Veröffentlichungstag und Bekanntmachung des Hinweises auf die Patenterteilung: **01.04.2026**
(21) Anmeldenummer: 25156523.0
(22) Anmeldetag: 07.02.2025
(51) Int. Cl.: A61M 1/16

(54) **DIALYSEEINHEIT UND VERFAHREN ZUM VORBEREITEN EINER DIALYSETHERAPIE**
DIALYSIS UNIT AND METHOD FOR PREPARING A DIALYSIS THERAPY
UNITÉ DE DIALYSE ET PROCÉDÉ DE PRÉPARATION D'UNE THÉRAPIE DE DIALYSE

(30) Priorität: 12.02.2024 DE 102024103806
(43) Veröffentlichungstag der Anmeldung: 13.08.2025
(73) Patentinhaber: B. Braun Avitum AG, 34212 Melsungen (DE)
(72) Erfinder: NIEMETZ, Guenter, 34212 Melsungen (DE)
(74) Vertreter: Winter, Brandl - Partnerschaft mbB

(56) Entgegenhaltungen:
- EP-B1- 0 622 086
- DE-A1- 1 566 662
- DE-A1- 102016 013 875

## Beschreibung

### Technisches Gebiet

Die vorliegende Offenbarung betrifft eine Dialyseeinheit, welche zumindest eine Dialysemaschine, insbesondere zur extrakorporalen Blutbehandlung eines Patienten, mit einer Dialysierflüssigkeitsvorbereitungsvorrichtung, welche dafür ausgebildet ist, eine Dialysierflüssigkeit für Dialysetherapien vor- / aufzubereiten, und zentrale Dialysierflüssigkeitskonzentrat-Versorgungseinrichtungen aufweist. Außerdem betrifft die Offenbarung ein Verfahren zum Vorbereiten einer Dialysetherapie.

### Hintergrund der Offenbarung

Dialyseeinheiten weisen herkömmlicherweise eine Vielzahl an Dialysemaschinen auf, die jeweils über Anschlussleitungen mit zentralen Dialysierflüssigkeitskonzentrat-Versorgungseinrichtungen verbunden sind, welche Dialysierflüssigkeitskonzentrate unterschiedlicher Konzentrationen enthalten, aus welchen die Dialysemaschinen diese beziehen und für eine nachfolgende Therapie Dialysierflüssigkeit/ -lösung in Dialysierflüssigkeitsvorbereitungsvorrichtungen vor-/ aufbereiten. In der chronischen Hämodialyse-Therapie werden pro Behandlung circa 120 bis 180 Liter Dialysierflüssigkeit/ -lösung benötigt. Diese Dialysierflüssigkeit wird herkömmlicherweise in der Dialysemaschine durch Verdünnung und Mischung eines basischen und eines sauren Dialysierflüssigkeitskonzentrats mit Osmosewasser/ Permeat hergestellt. Üblicherweise werden zentrale Dialysierflüssigkeitskonzentrat-Versorgungseinrichtungen mit großen Tanks eingesetzt. Mithilfe einer Pumpe wird ein Dialysierflüssigkeitskonzentrat über eine Ringleitung zu einem Behandlungsplatz gefördert.

Je nach Therapie unterscheiden sich die gewünschten Konzentrationen der Dialysierflüssigkeiten, weshalb während der Dialysetherapien einzelne (Anschluss-)Leitungen zum Befördern der Dialysierflüssigkeitskonzentrate stillstehen bzw. sich in einem inaktiven Zustand befinden. Üblicherweise bestehen diese Leitungen aus einem Kunststoffmaterial, weshalb bei einem Stillstand des entsprechenden Dialysierflüssigkeitskonzentrats in der entsprechenden Leitung Restwasser aus dem Dialysierflüssigkeitskonzentrat herausdiffundieren kann.

Individuelle Patientenbedürfnisse können eine Zuführung eines speziellen Dialysierflüssigkeitskonzentrats aus einem Dialysierflüssigkeitskonzentrat-Kanister, welcher extern an die entsprechende Dialysemaschine, bspw. über einen Ansaugstab, angeschlossen werden kann, erfordern. Aus diesem Grund können die Anschlussleitungen zwischen den jeweiligen Dialysemaschinen und den zentralen Dialysierflüssigkeitskonzentrat-Versorgungseinrichtungen gegebenenfalls auch für längere Zeiten stillstehen.

Die Dialysierflüssigkeitskonzentrate in den Dialysierflüssigkeitskonzentrat-Versorgungseinrichtungen sind von ihrer Zusammensetzung üblicherweise sehr ähnlich, d.h. die Komponentenzusammensetzung (bspw. NaCl, Mg, Ca) sind üblicherweise gleich. Unterschiedlich ist dann meist die Kalium-Konzentration der Dialysierflüssigkeitskonzentrate. Zum Beispiel können das erste Dialysierflüssigkeitskonzentrat eine Kalium-Konzentration von 1 mmol, das zweite Dialysierflüssigkeitskonzentrat eine Kalium-Konzentration von 2 mmol und der Kanister eine Sonderkonzentration von 4 mmol aufweisen. Werden dann beispielsweise 75% der Dialysetherapien mit dem Dialysierflüssigkeitskonzentrat mit 2mmol Kalium durchgeführt, dann würde das Dialysierflüssigkeitskonzentrat (mit einer unterschiedlichen Kalium-Konzentration) in der Anschlussleitung der anderen zentralen Dialysierflüssigkeitskonzentrat-Versorgungseinrichtung zu 75% der Zeit stillstehen bzw. die Anschlussleitung würde zu 75% der Zeit nicht durchspült/ durchflossen werden. Die daraus resultierende Konzentrationserhöhung (der Elektrolyte), vor allem bei längerem Stillstand der Leitungen, führt somit zu einer Kristallbildung in den (Anschluss-)Leitungen der Dialyseeinheit. Diese Kristalle sind dann meist nur mittels aufwändigen Reinigungs-/Wartungsprozessen entfernbar.

Des Weiteren wäre ein Durchspülen der Leitungen mit Permeat nachteilig, da Leitungen für Dialysierflüssigkeitskonzentrate herkömmlicherweise im Dead-End zu den Dialysemaschinen betrieben werden. Deshalb müssten dann alle vorgesehenen Dialysemaschinen einen Spül- und Füllprozess unterstützen. Der Kostenfaktor beim Spülen und Füllen wäre dabei nicht zu vernachlässigen, insbesondere da eine übermäßig große Menge von Dialysierflüssigkeitskonzentrat verworfen werden müsste.

Die DE 10 2016 013 875 A1 offenbart beispielsweise ein Reinigungsverfahren einer Dialyseeinheit mit einem zusätzlichen Spülvorgang der Dialysierflüssigkeitskonzentrate führenden (Anschluss-)Leitungen während eines Desinfektionsvorgangs des Dialysegerätes, welcher nach dem eigentlichen Therapieverfahren stattfindet.

Allerdings hat sich ein solcher Spülvorgang während des Desinfektionsvorgangs des Dialysegerätes als nachteilig herausgestellt. Insbesondere führt das zusätzliche Ansaugen von Dialysierflüssigkeitskonzentraten während der Gerätedesinfektion zu einer Zeitverlängerung des Desinfektionsprozesses, da Dialysierflüssigkeitskonzentrate und Desinfektionsmittel aufgrund von chemischen Unverträglichkeiten nicht gemeinsam in den Hydraulik-Leitungen des Dialysegerätes zirkulieren sollten. Zudem führt dies in nachteiliger Weise zu einer erhöhten Software-Komplexität der Dialyseeinheit bzw. einer Steuereinheit der Dialyseeinheit bzw. einer Steuereinheit der Dialysemaschine, da während des Desinfektionsprozesses Ausnahmen zu implementieren sind, welche ein Ansaugen der Dialysierflüssigkeitskonzentrate zulassen und gleichzeitig ein Zurückfließen dieser in deren jeweilige zentralen Dialysierflüssigkeitskonzentrat-Versorgungseinheiten verhindern.

### Kurzbeschreibung der vorliegenden Offenbarung

Daher liegt der vorliegenden Offenbarung die Aufgabe zugrunde, die vorstehend beschriebenen Nachteile zu vermeiden oder wenigstens abzumildern und insbesondere eine Dialyseeinheit bereitzustellen, welche einer Kristallbildung in einer Anschlussleitung zwischen einer Dialysemaschine und einer zentralen Dialysierflüssigkeitskonzentrat-Versorgungseinrichtung auf einfache und effiziente Weise entgegenwirkt. Weiterhin soll ein Verfahren zum Vorbereiten einer Dialysetherapie, welches beginnt, nachdem eine Dialysemaschine ohne einen daran angeschlossenen Patienten eingeschaltet wurde oder nachdem ein Patient von einer Dialysemaschine getrennt wurde, und welches endet, bevor ein Patient an die Dialysemaschine angeschlossen wird, also insbesondere ein Spülen bzw. Desinfizieren der Dialysemaschine, ein Warten auf ein Vorbereiten der Dialysierflüssigkeit und ein Vorbereiten der Dialysierflüssigkeit umfasst, nicht unnötig in die Länge gezogen werden.

Diese Aufgabe wird durch eine Dialyseeinheit gemäß den Merkmalen des Anspruchs 1, durch ein Verfahren zum Vorbereiten einer Dialysetherapie gemäß den Merkmalen des Anspruchs 13 und durch ein Computerprogramm gemäß den Merkmalen des Anspruchs 16 gelöst. Vorteilhafte Ausführungsformen sind in den Unteransprüchen beansprucht und/oder werden nachfolgend erläutert.

Die Offenbarung betrifft demzufolge zunächst eine Dialyseeinheit mit zumindest einer Dialysemaschine, welche eine Dialysierflüssigkeitsvorbereitungsvorrichtung / Dialysierflüssigkeitsaufbereitungsvorrichtung aufweist, die vorgesehen und eingerichtet ist, um eine Dialysierflüssigkeit/ Dialysierlösung für eine nachfolgende Dialysetherapie vorzubereiten, einer ersten zentralen Dialysierflüssigkeitskonzentrat-Versorgungseinrichtung, welche mit der Dialysemaschine über eine erste Anschlussleitung/ Versorgungsleitung verbunden ist, (zumindest) einer zweiten zentralen Dialysierflüssigkeitskonzentrat-Versorgungseinrichtung, welche mit der Dialysemaschine über eine zweite Anschlussleitung/ Versorgungsleitung verbunden ist, wobei die ersten und zweiten zentralen Dialysierflüssigkeitskonzentrat-Versorgungseinrichtungen unterschiedliche Dialysierflüssigkeitskonzentrate enthalten und der Dialysierflüssigkeitsvorbereitungsvorrichtung der Dialysemaschine bereitstellen können. Dabei weist die Dialyseeinheit, insbesondere die zumindest eine Dialysemaschine der Dialyseeinheit, eine Steuereinheit auf, welche eingerichtet ist, beim Vorbereiten der Dialysierflüssigkeit für die nachfolgende Dialysetherapie ein in dieser nicht zu verwendendes Dialysierflüssigkeitskonzentrat oder in dieser nicht zu verwendende Dialysierflüssigkeitskonzentrate aus der ersten zentralen Dialysierflüssigkeitskonzentrat-Versorgungseinrichtung und/oder der zweiten zentralen Dialysierflüssigkeitskonzentrat-Versorgungseinrichtung in die Dialysierflüssigkeitsvorbereitungsvorrichtung einzubringen und für eine Mischung / Aufbereitung der Dialysierflüssigkeit zu verwenden, um die erste Anschlussleitung und/oder die zweite Anschlussleitung zu spülen / zu durchfließen, und erst im Anschluss ein in der nachfolgenden Therapie zu verwendendes Dialysierflüssigkeitskonzentrat in die Dialysierflüssigkeitsvorbereitungsvorrichtung einzubringen und für die Mischung der Dialysierflüssigkeit zu verwenden.

In anderen Worten ausgedrückt ist die Steuereinheit der Dialyseeinheit/ der Dialysemaschine derart konfiguriert, beim Vorbereiten der Dialysierflüssigkeit für die nachfolgende Therapie einen (kurzen) Spülvorgang der Anschlussleitung(en)/ Versorgungsleitung(en) durchzuführen, welche in der anschließenden Dialysetherapie nicht verwendet werden. Der Spülvorgang erfolgt somit während der Auf-/ bzw. Vorbereitungsphase der Dialysierflüssigkeit, d.h. während der Leitfähigkeitsaufbereitung in einer Vorbereitungsphase für eine Dialysetherapie, und somit vorzugsweise nicht während dem Spülen bzw. der Desinfektion nach einer Dialysetherapie. Bevor die Dialysetherapie beginnen kann, werden üblicherweise verschiedene Selbsttests durchgeführt, wird der extrakorporale Kreislauf geprimed und wird der Auf- bzw. Vorbereitungsprozess der Dialysierflüssigkeit gestartet, d.h. das entsprechende Dialysierflüssigkeitskonzentrat wird bzw. die entsprechenden Dialysierflüssigkeitskonzentrate werden angesaugt, mit Osmosewasser vermischt und die (gemischte) Dialysierflüssigkeit wird anschließend temperiert. Da zu dieser Zeit noch kein Patient an die Dialysemaschine der Dialyseeinheit angeschlossen ist, kann gleich zu Beginn des Aufbereitungsprozesses der Dialysierflüssigkeit aus zumindest einer zentralen Dialysierflüssigkeitskonzentrat-Versorgungseinrichtung, vorzugsweise nacheinander oder gleichzeitig aus allen zentralen Dialysierflüssigkeitskonzentrat-Versorgungseinrichtungen, die in der nachfolgenden Dialysetherapie nicht zu verwendende Dialysierflüssigkeitskonzentrate enthalten, über die jeweiligen Anschlussleitungen eine beispielsweise dem Volumen der Anschlussleitungen entsprechende Menge Dialysierflüssigkeitskonzentrat angesaugt bzw. abgepumpt werden, damit sich wieder frisches Dialysierflüssigkeitskonzentrat in der entsprechenden Anschlussleitung bzw. den entsprechenden Anschlussleitungen befindet, welches nicht sofort zum Auskristallisieren neigt. Erst nach diesem vorhergehenden Spülvorgang wird offenbarungsgemäß das dem Patienten verschriebene bzw. einprogrammierte Dialysierflüssigkeitskonzentrat angesaugt und für die Aufbereitung der Dialysierflüssigkeit verwendet/ vermischt.

Bei der Leitfähigkeitsaufbereitung vor dem eigentlichen Dialysetherapieverfahren kann also offenbarungsgemäß ein Entscheidungsprozess der Steuereinheit stehen, welcher im Nachfolgenden beschrieben wird. Ist ein Kanister als Quelle des Dialysierflüssigkeitskonzentrats für die nachfolgende Dialysetherapie ausgewählt, werden bevorzugt die Anschlussleitungen aller, beispielsweise beider, zentralen Dialysierflüssigkeitskonzentrat-Versorgungseinrichtungen gespült, da in der nachfolgenden Therapie nur das Dialysierflüssigkeitskonzentrat aus dem Kanister zur Herstellung/ Anmischung/ Aufbereitung der Dialysierflüssigkeit verwendet wird, wodurch die Anschlussleitungen aller, beispielsweise beider, zentralen Dialysierflüssigkeitskonzentrat-Versorgungseinrichtungen für die gesamte Dauer des Prozesses inaktiv bleiben, d.h. es würde kein Fluid durch diese befördert werden. Ist das Dialysierflüssigkeitskonzentrat aus der ersten zentralen Dialysierflüssigkeitskonzentrat-Versorgungseinrichtung ausgewählt, wird demnach bevorzugt im Falle von zwei zentralen Dialysierflüssigkeitskonzentrat-Versorgungseinrichtungen nur die Anschlussleitung der zweiten zentralen Dialysierflüssigkeitskonzentrat-Versorgungseinrichtung gespült, da in der nachfolgenden Therapie das Dialysierflüssigkeitskonzentrat über die Anschlussleitung der ersten zentralen Dialysierflüssigkeitskonzentrat-Versorgungseinrichtung zur Dialysierflüssigkeitsherstellung verwendet wird. Dementsprechend wird bevorzugt im Falle von zwei zentralen Dialysierflüssigkeitskonzentrat-Versorgungseinrichtungen nur die Anschlussleitung der ersten zentralen Dialysierflüssigkeitskonzentrat-Versorgungseinrichtung gespült, wenn in der nachfolgenden Therapie das Dialysierflüssigkeitskonzentrat über die Anschlussleitung der zweiten zentralen Dialysierflüssigkeitskonzentrat-Versorgungseinrichtung zur Dialysierflüssigkeitsherstellung verwendet wird.

In nochmals anderen Worten ausgedrückt, kann, wenn für die nachfolgende (Dialyse-)Therapie Dialysierflüssigkeitskonzentrat aus der ersten zentralen Dialysierflüssigkeitskonzentrat-Versorgungseinrichtung verordnet ist, das Anmischen und Einregeln der Dialysierflüssigkeit mit Dialysierflüssigkeitskonzentrat aus der zweiten zentralen Dialysierflüssigkeitskonzentrat-Versorgungseinrichtung beginnen. Nachdem das Spülvolumen der betroffenen Anschlussleitung erreicht ist, kann die Dialyseeinheit/ das System/ die vorgesehene Steuereinheit automatisch auf das Dialysierflüssigkeitskonzentrat aus der ersten zentralen Dialysierflüssigkeitskonzentrat-Versorgungseinrichtung schalten und die Aufbereitung vervollständigen. Wenn für die Therapie Dialysierflüssigkeitskonzentrat aus der zweiten Dialysierflüssigkeitskonzentrat-Versorgungseinrichtung verordnet ist, kann das Anmischen und Einregeln mit der ersten Dialysierflüssigkeitskonzentrat-Versorgungseinrichtung beginnen. Nachdem das Spülvolumen dieser Anschlussleitung erreicht ist, kann die Dialyseeinheit automatisch auf das Dialysierflüssigkeitskonzentrat aus der zweiten zentralen Dialysierflüssigkeitskonzentrat-Versorgungseinrichtung schalten und die Aufbereitung vervollständigen. Wenn für die Therapie Dialysierflüssigkeitskonzentrat aus einem externen Kanister verordnet ist, kann das Anmischen und Einregeln mit Dialysierflüssigkeitskonzentrat aus der ersten zentralen Dialysierflüssigkeitskonzentrat-Versorgungseinrichtung erfolgen und nachdem das Spülvolumen der ersten Anschlussleitung zwischen der Dialysemaschine und der ersten zentralen Dialysierflüssigkeitskonzentrat-Versorgungseinrichtung erreicht ist, kann die Steuereinheit automatisch auf das Dialysierflüssigkeitskonzentrat der zweiten zentralen Dialysierflüssigkeitskonzentrat-Versorgungseinrichtung umschalten und den Anmisch- und Einregelprozess mit dem Spülvolumen des Dialysierflüssigkeitskonzentrats aus der zweiten zentralen Dialysierflüssigkeitskonzentrat-Versorgungseinrichtung fortsetzen. Alternativ kann das Spülen der ersten und zweiten Anschlussleitungen auch gleichzeitig erfolgen. Nachdem das Spülvolumen von sowohl der ersten Anschlussleitung als auch der zweiten Anschlussleitung zwischen der Dialysemaschine und den ersten und zweiten zentralen Dialysierflüssigkeitskonzentrat-Versorgungseinrichtungen erreicht ist, kann die vorgesehene Steuereinheit (der Dialysemaschine) automatisch auf das Dialysierflüssigkeitskonzentrat aus dem Kanister umschalten und die Aufbereitung der Dialysierflüssigkeit für die nachfolgende Dialysetherapie vervollständigen.

Das Spülvolumen der Dialysierflüssigkeitskonzentrate beträgt im Allgemeinen einige Milliliter und wird beim Anmischprozess nach wenigen Sekunden erreicht. Üblicherweise dauert der gesamte Anmisch- und Einregelprozess der Dialysierflüssigkeit einige Minuten, sodass die Spülmengen der Leitungen bereits im Abfluss sind, bis das System mit der korrekt aufbereiteten Dialysierflüssigkeit einen anzuschließenden Patienten therapiert. Vorteilhafterweise ermöglicht eine derartig ausgebildete Dialyseeinheit ein automatisches Spülen der Anschlussleitungen, um einer Kristallbildung durch das stillstehende Dialysierflüssigkeitskonzentrat in den Leitungen entgegenzuwirken, und zwar insbesondere ohne einen signifikanten Zeitmehraufwand bei dem Vorbereiten der Therapie, welches insbesondere die Schritte: Spülen bzw. Desinfizieren; Warten auf Vorbereiten der Dialysierflüsigkeit; und Vorbereiten der Dialysierflüssigkeit umfasst, insbesondere bei dem Schritt "Vorbereiten der Dialysierflüssigkeit" zu verursachen. Demnach ist der Spülvorgang der Anschlussleitungen in den Vorbereitungsprozess vor einer Dialysetherapie mit einbezogen, um möglichst wenig zusätzliche Zeit für diesen Prozess einplanen zu müssen.

Wenn gemäß der vorliegenden Offenbarung die ersten und zweiten zentralen Dialysierflüssigkeitskonzentrat-Versorgungseinrichtungen unterschiedliche Dialysierflüssigkeitskonzentrate enthalten, bedeutet dies bevorzugt, dass die Unterschiede eher geringfügiger Natur sind. Insbesondere kann es vorgesehen sein, dass die Dialysierfüssigkeitskonzentrate in den zentralen Dialysierflüssigkeitskonzentrat-Versorgungseinrichtungen von ihrer (Komponenten-) Zusammensetzung identisch sind. Besonders bevorzugt unterscheiden sich lediglich die Kalium-Konzentrationen der Dialysierflüssigkeitskonzentrate in den zentralen Dialysierflüssigkeitskonzentrat-Versorgungseinrichtungen. Zum Beispiel können das erste Dialysierflüssigkeitskonzentrat eine Kalium-Konzentration von 1 mmol und das zweite Dialysierflüssigkeitskonzentrat eine Kalium-Konzentration von 2 mmol aufweisen. Insbesondere vor dem Hintergrund der ähnlichen, sich lediglich geringfügig unterscheidenden Dialysierflüssigkeitskonzentrate kann es offenbarungsgemäß vorgesehen sein, zunächst kurz eine Anmischung der Dialysierflüssigkeit mit einem "falschen" Dialysierflüssigkeitskonzentrat vorzunehmen.

Die Steuereinheit kann grundsätzlich eingerichtet sein, bei jedem Vorbereiten der Dialysierflüssigkeit für die nachfolgende Dialysetherapie das Spülen der entsprechenden Anschlussleitung bzw. der entsprechenden Anschlussleitungen, also der Anschlussleitung(en), die Dialysierflüssigkeitskonzentrat(e) führt bzw. führen, welche(s) in der nachfolgenden Therapie nicht zu verwenden ist bzw. sind, durchzuführen.

Alternativ kann die Steuereinheit eingerichtet sein, eine Inaktivität / einen Stillstand der (ersten und zweiten) Anschlussleitungen zeitlich zu überwachen und die erste und/oder die zweite Anschlussleitung nach Erreichen/ beim Überschreiten einer vorbestimmten Zeitdauer der Inaktivität mit dem Dialysierflüssigkeitskonzentrat zu spülen. In diesem Fall kann die Steuereinheit somit eingerichtet sein, nicht bei jedem Vorbereiten der Dialysierflüssigkeit für die nachfolgende Dialysetherapie, sondern erst nachdem die vorbestimmte Zeitdauer der Inaktivität verstrichen ist, das Spülen der entsprechenden Anschlussleitung bzw. der entsprechenden Anschlussleitungen durchzuführen.

Anders ausgedrückt kann (zusätzlich) eine zeitliche Überwachung durch die Steuereinheit erfolgen und wenn eine der Anschlussleitungen (oder beide) länger als eine vorbestimmte Zeit nicht benutzt wurde, kann im Vorbereiten der nächsten Therapie ein Spülvorgang für diese Anschlussleitung durchgeführt/ initiiert werden.

Dies hat den Vorteil, dass sichergestellt ist, dass die Anschlussleitungen ausreichend durchspült werden, auch für den Fall, dass eine Stillstands- / Wartephase zwischen zwei Dialysetherapien eines Patienten eine längere Zeitdauer in Anspruch nehmen sollte. Dies stellt eine zusätzliche Sicherheit dar, ein Auskristallisieren von Dialysierflüssigkeitskonzentrat in den Leitungen zu verhindern, unabhängig davon, wie lange die Dialyseeinheit zwischen zwei Therapieschleifen stillsteht. Darüber hinaus kann dadurch ein möglicherweise unnötiges Spülen einer Anschlussleitung vermieden werden, beispielsweise, wenn ein Dialysierflüssigkeitskonzentrat aus einer zentralen Dialysierflüssigkeitskonzentrat-Versorgungseinrichtung, das in der nachfolgenden Dialysetherapie nicht verwendet wird, zeitlich unmittelbar vorher in der vorhergehenden Dialysetherapie verwendet wurde.

**In** einer weiteren bevorzugten Ausführungsform der Offenbarung weisen die zentralen Dialysierflüssigkeitskonzentrat-Versorgungseinrichtungen jeweils ein Dialysierflüssigkeitskonzentrat-Reservoir/-Tank zum Aufbewahren/ Speichern der Dialysierflüssigkeitskonzentrate und eine daran angeschlossene Ringleitung auf. Die Ringleitungen sind dafür ausgebildet, um die jeweiligen Dialysierflüssigkeitskonzentrate zu den Behandlungsplätzen zu befördern. Weiter bevorzugt sind die Anschlussleitungen über Ringleitungskonnektoren, insbesondere lösbar, mit den Ringleitungen und über Dialysemaschinenkonnektoren, insbesondere lösbar, mit der Dialysemaschine verbunden. Somit sind die zentralen Dialysierflüssigkeitskonzentrat-Versorgungseinrichtungen in Abhängigkeit der gewünschten Dialysierflüssigkeitskonzentrate an eine Dialysemaschine koppelbar und im Nachhinein auch wieder von dieser trennbar.

**In** einem weiteren vorteilhaften Aspekt der Offenbarung stehen die erste Anschlussleitung mit einer ersten Verbindungsleitung und die zweite Anschlussleitung mit einer zweiten Verbindungsleitung in Verbindung. Die Verbindungsleitungen sind dabei innerhalb der Dialysemaschine angeordnet und die Dialysierflüssigkeitskonzentrate aus den zentralen Dialysierflüssigkeitskonzentrat-Versorgungseinrichtungen sind über die Anschlussleitungen und die Verbindungsleitungen der Dialysierflüssigkeitsvorbereitungsvorrichtung zuführbar.

Vorzugsweise weisen die Verbindungsleitungen jeweils ein erstes Einlassventil, ein zweites Einlassventil und einen Drucksensor/ Druckermittler auf und die Steuereinheit der Dialysemaschine ist eingerichtet, über die Drucksensoren einen (Über-)Druck in den ersten und zweiten Verbindungsleitungen zu erfassen und dadurch zu bestimmen, ob die jeweiligen Anschlussleitungen an die Dialysemaschine angeschlossen sind.

Anders ausgedrückt ist die Dialyseeinheit derart ausgebildet, dass die Dialysierflüssigkeitskonzentrate mit einem Überdruck durch die Ringleitungen der zentralen Dialysierflüssigkeitskonzentrat-Versorgungseinrichtungen beförderbar oder befördert sind. Dadurch kann durch ein Öffnen des ersten Einlassventils bei gleichzeitig geschlossenem zweiten Einlassventil einer Verbindungsleitung über den zwischen diesen Ventilen angeordneten Drucksensor festgestellt werden, ob eine zentrale Dialysierflüssigkeitskonzentrat-Versorgungseinrichtung über eine Anschlussleitung an die Dialysemaschine angeschlossen ist oder nicht. Nur wenn dadurch ein Überdruck ermittelt wird, wird eine Anschlussleitung bevorzugt auch wirklich gespült. Wird an einem Drucksensor kein Druck gemessen, bedeutet das, dass keine zentrale Dialysierflüssigkeitskonzentrat-Versorgungseinrichtung über die jeweilige Anschlussleitung angeschlossen ist, weshalb ein Spülvorgang bevorzugt gar nicht erst durchgeführt wird. Die Leitungsanschlüsse werden also auf Konnektierung/ Anschlüsse überprüft. Dadurch wird der Zeitaufwand für Spülvorgänge der Anschlussleitungen optimiert und zusätzlich geringgehalten.

Allgemeiner ausgedrückt ist die vorgesehene Steuereinheit somit bevorzugt eingerichtet, insbesondere über einen Drucksensor/ Druckermittler festzustellen, ob eine oder mehrere zentrale Dialysierflüssigkeitskonzentrat-Versorgungseinrichtungen an die Dialysemaschine angeschlossen bzw. konnektiert sind.

Der Drucksensor/ Druckermittler kann in der Anschlussleitung zwischen der Dialysemaschine und der zentralen Dialysierflüssigkeitskonzentrat-Versorgungseinrichtung vorgesehen sein. Alternativ und bevorzugt kann der Drucksensor/ Druckermittler in der Verbindungsleitung innerhalb der Dialysemaschine vorgesehen sein.

Dem Drucksensor/ Druckermittler kann ein (Einlass-)Ventil nachgeschaltet sein. Bevorzugt ist die Steuereinheit eingerichtet, das dem Drucksensor/ Druckermittler nachgeschaltete (Einlass-)Ventil für ein Spülen der Anschlussleitung zu öffnen, wenn sie feststellt bzw. festgestellt hat, dass eine zentrale Dialysierflüssigkeitskonzentrat-Versorgungseinrichtungen an die Dialysemaschine angeschlossen bzw. konnektiert ist, welche in der nachfolgenden Dialysetherapie nicht zu verwendendes Dialysierflüssigkeitskonzentrat führt.

Dem Drucksensor/ Druckermittler kann ferner ein (Einlass-)Ventil vorgeschaltet sein. Bevorzugt ist die Steuereinheit eingerichtet, das dem Drucksensor/ Druckermittler vorgeschaltete (Einlass-)Ventil zu öffnen, um über den Drucksensor/ Druckermittler festzustellen, ob eine zentrale Dialysierflüssigkeitskonzentrat-Versorgungseinrichtung an die Dialysemaschine angeschlossen bzw. konnektiert ist.

Allgemeiner ausgedrückt ist die vorgesehene Steuereinheit somit bevorzugt eingerichtet, wenn bzw. nachdem sie feststellt bzw. festgestellt hat, dass eine oder mehrere zentrale Dialysierflüssigkeitskonzentrat-Versorgungseinrichtungen an die Dialysemaschine angeschlossen bzw. konnektiert sind, beim Vorbereiten der Dialysierflüssigkeit für die nachfolgende Dialysetherapie ein in dieser nicht zu verwendendes Dialysierflüssigkeitskonzentrat bzw. in dieser nicht zu verwendende Dialysierflüssigkeitskonzentrate aus der ersten zentralen Dialysierflüssigkeitskonzentrat-Versorgungseinrichtung und/oder der zweiten zentralen Dialysierflüssigkeitskonzentrat-Versorgungseinrichtung in die Dialysierflüssigkeitsvorbereitungsvorrichtung einzubringen und für eine Mischung / Aufbereitung der Dialysierflüssigkeit zu verwenden, um die erste Anschlussleitung und/oder die zweite Anschlussleitung zu spülen / zu durchfließen, und erst im Anschluss ein in der nachfolgenden Therapie zu verwendendes Dialysierflüssigkeitskonzentrat in die Dialysierflüssigkeitsvorbereitungsvorrichtung einzubringen und für die Mischung der Dialysierflüssigkeit zu verwenden.

In einem weiteren vorteilhaften Aspekt der Offenbarung ist die Steuereinheit eingerichtet, die erste und/oder die zweite Anschlussleitung mit einem minimal zum Spülen der Anschlussleitungen erforderlichen Spülvolumen der Dialysierflüssigkeitskonzentrate zu spülen, welches dem Volumen der ersten und/oder der zweiten Anschlussleitung entspricht. Ein entsprechender Wert für ein solches erforderliches Spülvolumen kann in einem Speicher der Dialysemaschine jeweils für die entsprechende Anschlussleitung hinterlegt sein.

In einer weiteren bevorzugten Ausführungsform der Offenbarung ist die Steuereinheit eingerichtet, das notwendige/ minimal erforderliche Spülvolumen der Dialysierflüssigkeitskonzentrate zum Spülen der Anschlussleitungen aus einem Schlauchdurchmesser, bspw. 5mm, und einer Schlauchlänge, bspw. 2m, der jeweiligen Anschlussleitungen vorzubestimmen/ zu berechnen. Anders ausgedrückt ist die Steuereinheit in der Lage, mithilfe von Informationen über die Geometrie/ Abmessungen der Anschlussleitungen ein minimales Spülvolumen zu bestimmen bzw. orientiert sich die Steuereinheit bevorzugt zur Berechnung an diesen Informationen, damit die entsprechenden Anschlussleitungen ausreichend durchspült werden. Je genauer das Spülvolumen bestimmt werden kann, desto kürzer können die Spülvorgänge der Leitungen sein und es kann sichergestellt werden, dass die Dialysierflüssigkeitskonzentrate zum Durchspülen bereits im Abfluss sind, bis das System später mit der korrekt aufbereiteten Dialysierflüssigkeit den anzuschließenden Patienten therapiert. Die Steuereinheit ist entsprechend bevorzugt auch eingerichtet, das berechnete bzw. bestimmte Spülvolumen insbesondere über eine vorgesehene Dialysierflüssigkeitskonzentratpumpe in die Dialysierflüssigkeitsvorbereitungsvorrichtung einzubringen bzw. anzusaugen.

In einer weiteren vorteilhaften Ausführungsform der Offenbarung weist die Dialyseeinheit zumindest eine, beispielsweise zwei, drei, vier, fünf, sechs oder mehr, weitere Dialysemaschinen auf, welche mit den zentralen Dialysierflüssigkeitskonzentrat-Versorgungseinrichtungen über eine erste und eine zweite zusätzliche Anschlussleitung verbunden ist bzw. sind. Dabei ist die zumindest eine weitere Dialysemaschine bzw. deren Steuereinheit eingerichtet, beim Vorbereiten der Dialysierflüssigkeit für die nachfolgende Dialysetherapie die erste und/oder die zweite zusätzliche Anschlussleitung gemäß der offenbarungsgemäßen technischen Lehre durchzuspülen.

Weiter bevorzugt können mehr als zwei zentrale Dialysierflüssigkeitskonzentrat-Versorgungseinrichtungen, beispielsweise drei, vier, fünf, sechs oder mehr, zentrale Dialysierflüssigkeitskonzentrat-Versorgungseinrichtungen vorgesehen sein, welche jeweils über Anschlussleitungen an die Dialysemaschine angeschlossen sind. Bevorzugt ist die Steuereinheit eingerichtet, beim Vorbereiten der Dialysierflüssigkeit für die nachfolgende Dialysetherapie mehrere oder alle in dieser nicht zu verwendende Dialysierflüssigkeitskonzentrate aus den angeschlossenen zentralen Dialysierflüssigkeitskonzentrat-Versorgungseinrichtungen in die Dialysierflüssigkeitsvorbereitungsvorrichtung einzubringen und für eine Mischung / Aufbereitung der Dialysierflüssigkeit zu verwenden, um die entsprechenden Anschlussleitungen zu spülen/ zu durchfließen, und erst im Anschluss ein in der nachfolgenden Therapie zu verwendendes Dialysierflüssigkeitskonzentrat in die Dialysierflüssigkeitsvorbereitungsvorrichtung einzubringen und für die Mischung der Dialysierflüssigkeit zu verwenden.

Jede der vorgesehenen Dialysemaschinen kann mit jeder der vorgesehenen zentralen Dialysierflüssigkeitskonzentrat-Versorgungseinrichtungen über Anschlussleitungen verbunden sein. Auch anders herum kann jede der zentralen Dialysierflüssigkeitskonzentrat-Versorgungseinrichtungen mit jeder der vorgesehenen Dialysemaschinen über Anschlussleitungen verbunden sein.

**In** einem weiteren bevorzugten Aspekt der Offenbarung weist die Dialysemaschine eine Dialysierflüssigkeitskonzentratpumpe auf, welche ausgebildet ist, die Dialysierflüssigkeitskonzentrate aus den (ersten und/oder zweiten) zentralen Dialysierflüssigkeitskonzentrat-Versorgungseinrichtungen in die Dialysierflüssigkeitsvorbereitungsvorrichtung zu befördern/ pumpen. Insbesondere ist diese Dialysierflüssigkeitskonzentratpumpe als eine Kolbenpumpe ausgeführt.

In einer weiteren vorteilhaften Ausführungsform der Offenbarung ist die Steuereinheit eingerichtet, das in der nachfolgenden Therapie zu verwendende Dialysierflüssigkeitskonzentrat durch die Dialysierflüssigkeitskonzentratpumpe über einen Ansaugstab aus einem Dialyseflüssigkeitskonzentrat-Kanister anzusaugen und für die Mischung der Dialysierflüssigkeit in der Dialysierflüssigkeitsvorbereitungsvorrichtung zu verwenden. Der Kanister kann somit extern an die Dialysemaschine angeschlossen sein und weist vorzugsweise ein Dialysierflüssigkeitskonzentrat mit einer speziellen Komponentenkonzentration auf, welches für individuelle Patientenbedürfnisse einsetzbar ist. Das Dialysierflüssigkeitskonzentrat des Kanisters wird also getrennt bzw. unabhängig von den Dialysierflüssigkeitskonzentraten in den zentralen Dialysierflüssigkeitskonzentrat-Versorgungseinrichtungen verwendet.

In einem weiteren bevorzugten Aspekt der Offenbarung ist die Dialysierflüssigkeitsvorbereitungsvorrichtung der Dialysemaschine ausgebildet, die Dialysierflüssigkeit für die nachfolgende Dialysetherapie aus einem (sauren) Dialysierflüssigkeitskonzentrat aus einer der zentralen Dialysierflüssigkeitskonzentrat-Versorgungseinrichtungen oder des externen Kanisters, aus hochreinem Wasser bevorzugt Osmosewasser aus einem Osmosetank oder einer zentralen RO-Anlage/Versorgungsleitung und aus einem weiteren (basischen) Dialysierflüssigkeitskonzentrat vorzubereiten. Anders ausgedrückt stellt die Dialysierflüssigkeitsvorbereitungsvorrichtung die Dialysierflüssigkeit/ -lösung, welche für die nachfolgende Dialysetherapie eingesetzt wird, durch das Mischen eines Dialysierflüssigkeitskonzentrats der zentralen Dialysierflüssigkeitskonzentrat-Versorgungseinrichtungen oder des externen Kanisters mit Osmosewasser als ein Permeat und vorzugsweise ferner mit einem basischen Dialysierflüssigkeitskonzentrat, welches extern also von außen in die Dialysemaschine eingeleitet wird/ einleitbar ist, bereit.

Die Offenbarung betrifft weiterhin ein Verfahren zum Vorbereiten einer Dialysetherapie, insbesondere zum Vorbereiten einer Dialysierflüssigkeit für eine nachfolgende Dialysetherapie, welches erst beginnt, nachdem die Dialysemaschine bzw. die Dialyseeinheit ohne einen daran angeschlossenen/ konnektierten Patienten eingeschaltet wurde oder nachdem ein Patient von einer Dialysemaschine getrennt wurde, und welches endet, bevor ein Patient an die Dialysemaschine angeschlossen wird, mit den Schritten: a) Einbringen eines in der nachfolgenden Dialysetherapie nicht zu verwendenden Dialysierflüssigkeitskonzentrats oder von in der nachfolgenden Dialysetherapie nicht zu verwendenden Dialysierflüssigkeitskonzentraten aus einer ersten zentralen Dialysierflüssigkeitskonzentrat-Versorgungseinrichtung und/oder einer zweiten zentralen Dialysierflüssigkeitskonzentrat-Versorgungseinrichtung in eine Dialysierflüssigkeitsvorbereitungsvorrichtung der Dialysemaschine und Verwenden des in der nachfolgenden Dialysetherapie nicht zu verwendenden Dialysierflüssigkeitskonzentrats oder der in der nachfolgenden Dialysetherapie nicht zu verwendenden Dialysierflüssigkeitskonzentrate für eine Mischung der Dialysierflüssigkeit; und erst im Anschluss, b) Einbringen eines in der nachfolgenden Dialysetherapie zu verwendenden Dialysierflüssigkeitskonzentrats in die Dialysierflüssigkeitsvorbereitungsvorrichtung der Dialysemaschine und Verwenden des in der nachfolgenden Dialysetherapie zu verwendenden Dialysierflüssigkeitskonzentrats für die Mischung der Dialysierflüssigkeit. Die Schritte a) und b) werden somit nacheinander, das heißt zunächst Schritt a) und danach Schritt b) durchgeführt.

Dieses Vorbereitungsverfahren wird also vor einer ersten Dialysetherapie nach einem Einschalten der Dialysemaschine ohne einen daran angeschlossenen/ konnektierten Patienten oder zwischen zwei Dialysetherapien durchgeführt, insbesondere nachdem ein Patient von der Dialysemaschine abgetrennt wurde und bevor ein Patient an die Dialysemaschine für eine nachfolgende Dialysetherapie angeschlossen wird und ist somit kein Verfahren zur chirurgischen oder therapeutischen Behandlung des menschlichen oder tierischen Körpers und kein Diagnostizierverfahren, das am menschlichen oder tierischen Körper vorgenommen wird.

Vorzugsweise weist das Verfahren einen Schritt c) auf, welcher bevorzugt vor Schritt a) vorgenommen wird: c) (Vor-)Bestimmen oder Berechnen oder Abrufen eines minimal erforderlichen Spülvolumens des in der nachfolgenden Dialysetherapie nicht zu verwendenden Dialysierflüssigkeitskonzentrats oder der in der nachfolgenden Dialysetherapie nicht zu verwendenden Dialysierflüssigkeitskonzentrate in Abhängigkeit eines Schlauchdurchmessers und einer Schlauchlänge einer Anschlussleitung, welche die zentralen Dialysierflüssigkeitskonzentrat-Versorgungseinrichtung mit der Dialysemaschine verbindet. Alternativ oder zusätzlich weist das Verfahren ferner einen Schritt d) auf: d) Einbringen des bestimmten oder berechneten oder abgerufenen (beispielsweise im Voraus in der Dialysemaschine hinterlegten oder als Systemwert abgespeicherten) Spülvolumens in Schritt a), insbesondere über eine vorgesehene Dialysierflüssigkeitskonzentratpumpe. Der entsprechende Wert für ein solches erforderliches Spülvolumen kann in einem Speicher der Dialysemaschine jeweils für die entsprechende Anschlussleitung hinterlegt sein. In anderen Worten kann ein Spülen der ersten Anschlussleitung und/oder der zweiten Anschlussleitung mit dem minimal erforderlichen Spülvolumen der Dialysierflüssigkeitskonzentrate, welches dem Volumen der ersten Anschlussleitung und/oder der zweiten Anschlussleitung entspricht, erfolgen.

Weiter bevorzugt weist das Verfahren einen Schritt e) auf, welcher bevorzugt vor Schritt a) vorgenommen wird: e) Feststellen, insbesondere über einen Drucksensor/ Druckermittler, ob eine oder mehrere zentrale Dialysierflüssigkeitskonzentrat-Versorgungseinrichtungen an die Dialysemaschine angeschlossen bzw. konnektiert sind. Bevorzugt wird Schritt a) nur bei angeschlossenen bzw. konnektierten zentralen Dialysierflüssigkeitskonzentrat-Versorgungseinrichtungen vorgenommen, also wenn in Schritt e) festgestellt wird, dass eine oder mehrere zentrale Dialysierflüssigkeitskonzentrat-Versorgungseinrichtungen an die Dialysemaschine angeschlossen bzw. konnektiert sind.

Das Verfahren kann ferner einen Schritt f) aufweisen: f) Überwachen einer Inaktivität/ eines Stillstands der Anschlussleitungen und Durchführen von Schritt a) erst, wenn eine vorbestimmte Zeitdauer der Inaktivität verstrichen ist. Alternativ kann Schritt a) auch grundsätzlich bei jedem Vorbereiten der Dialysierflüssigkeit durchgeführt werden.

Die Offenbarung betrifft weiterhin ein Computerprogramm, umfassend Befehle, die bei einer Ausführung durch einen Computer diesen veranlassen, die folgenden Verfahrensschritte auszuführen: a) Einbringen eines in einer nachfolgenden Dialysetherapie nicht zu verwendenden Dialysierflüssigkeitskonzentrats oder von in der nachfolgenden Dialysetherapie nicht zu verwendenden Dialysierflüssigkeitskonzentraten aus einer ersten zentralen Dialysierflüssigkeitskonzentrat-Versorgungseinrichtung und/oder einer zweiten zentralen Dialysierflüssigkeitskonzentrat-Versorgungseinrichtung in eine Dialysierflüssigkeitsvorbereitungsvorrichtung einer Dialysemaschine und Verwenden des in der nachfolgenden Dialysetherapie nicht zu verwendenden Dialysierflüssigkeitskonzentrats oder der in der nachfolgenden Dialysetherapie nicht zu verwendenden Dialysierflüssigkeitskonzentrate für eine Mischung der Dialysierflüssigkeit; und im Anschluss, b) Einbringen eines in der nachfolgenden Dialysetherapie zu verwendenden Dialysierflüssigkeitskonzentrats in die Dialysierflüssigkeitsvorbereitungsvorrichtung der Dialysemaschine und Verwenden des in der nachfolgenden Dialysetherapie zu verwendenden Dialysierflüssigkeitskonzentrats für die Mischung der Dialysierflüssigkeit.

### Kurzbeschreibung der Figuren

Die Offenbarung wird nachfolgend anhand vorteilhafter Ausführungsformen und unter Bezugnahme auf die dazugehörenden Figuren näher erläutert. Es zeigen:
Fig. 1 eine Dialyseeinheit in einer schematischen Darstellung mit zwei zentralen Dialysierflüssigkeitskonzentrat-Versorgungseinrichtungen und mindestens einer Dialysemaschine gemäß der vorliegenden Offenbarung; und
Fig. 2 ein Flussdiagramm eines Verfahrens zum Vorbereiten einer Dialysetherapie gemäß der vorliegenden Offenbarung.

Die Figuren sind lediglich schematischer Natur und dienen ausschließlich dem Verständnis der vorliegenden Offenbarung. Es wird darauf hingewiesen, dass die Merkmale der einzelnen Ausführungsformen untereinander ausgetauscht werden sowie in einer bestimmten Kombination auftreten können.

### Detaillierte Beschreibung bevorzugter Ausführungsformen

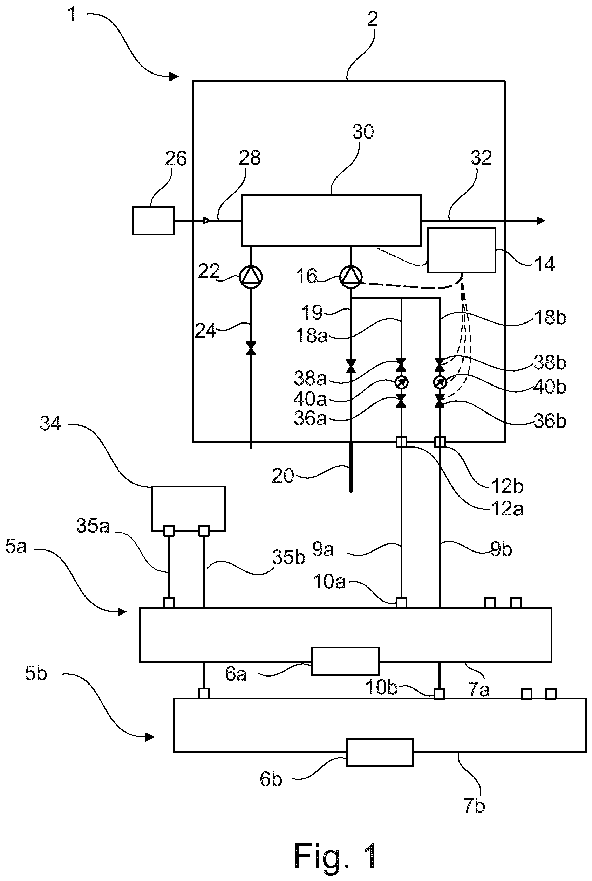

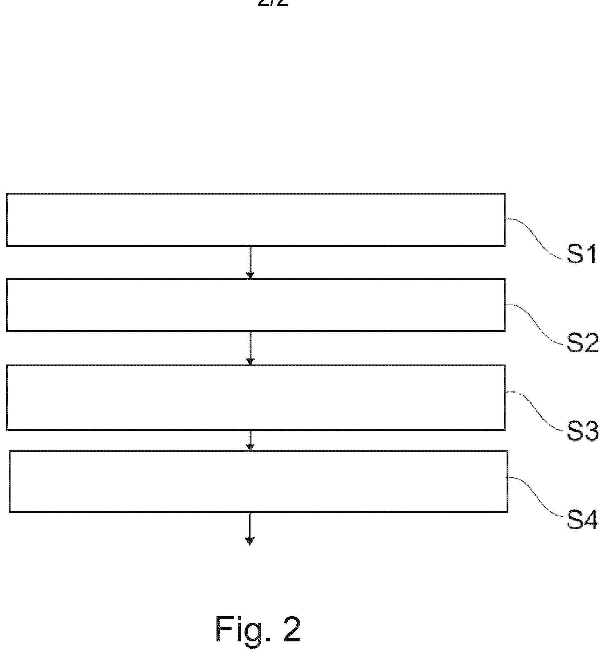

Nachfolgend wird die vorliegende Offenbarung anhand der bevorzugten Ausführungsform mit Bezug auf die Figuren 1 und 2 beschrieben. Dabei zeigt Figur 1 eine Dialyseeinheit 1 in einer schematischen Darstellung. Figur 2 zeigt ein Flussdiagramm eines beispielhaften Verfahrensablaufs zum Vorbereiten einer Dialysetherapie.

Figur 1 zeigt eine Dialyseeinheit 1 mit einer (ersten) Dialysemaschine 2 und einer ersten zentralen Dialysierflüssigkeitskonzentrat-Versorgungseinrichtung 5a sowie einer zweiten zentralen Dialysierflüssigkeitskonzentrat-Versorgungseinrichtung 5b. Die erste Dialysierflüssigkeitskonzentrat-Versorgungseinrichtung 5a weist ein erstes Dialysierflüssigkeitskonzentrat-Reservoir 6a, das ein erstes Dialysierflüssigkeitskonzentrat zur Herstellung einer Dialysierflüssigkeit aufbewahrt, und eine erste Ringleitung 7a auf, die mit dem ersten Dialysierflüssigkeitskonzentrat-Reservoir 6a in Verbindung steht. Gleichermaßen weist die zweite Dialysierflüssigkeitskonzentrat-Versorgungseinrichtung 5b ein zweites Dialysierflüssigkeitskonzentrat-Reservoir 6b mit einem zweiten Dialysierflüssigkeitskonzentrat und eine zweite Ringleitung 7b auf, die dieselbe Anordnung zueinander aufweisen wie die Komponenten der ersten Dialysierflüssigkeitskonzentrat-Versorgungseinrichtung 5a. Dabei weisen die Dialysierflüssigkeitskonzentrate beider Dialysierflüssigkeitskonzentrat-Versorgungseinrichtungen 5a, 5b unterschiedliche Zusammensetzungen bzw. Konzentrationen auf. Es können auch weitere (nicht dargestellte) zentrale Dialysierflüssigkeitskonzentrat-Versorgungseinrichtungen vorgesehen sein.

Die Dialysemaschine 2 steht dabei über eine erste Anschlussleitung 9a mit der ersten Dialysierflüssigkeitskonzentrat-Versorgungseinrichtung 5a und über eine zweite Anschlussleitung 9b mit der zweiten Dialysierflüssigkeitskonzentrat-Versorgungseinrichtung 5b in Verbindung. Auf Seiten der Dialysierflüssigkeitskonzentrat-Versorgungseinrichtungen 5a, 5b sind die Anschlussleitungen 9a, 9b jeweils über einen Ringleitungskonnektor 10a, 10b angeschlossen und auf Seiten der Dialysemaschine 2 sind die Anschlussleitungen 9a, 9b über Dialysemaschinenkonnektoren 12a, 12b mit dieser gekoppelt.

Die Dialysemaschine 2 weist eine Steuereinheit 14 und eine (erste) Dialysierflüssigkeitskonzentratpumpe 16 auf, welche über eine erste und eine zweite, insbesondere dialysemaschineninterne, Verbindungsleitung 18a, 18b mit den Anschlussleitungen 9a, 9b verbunden sind. Das heißt, die Anschlussleitungen 9a, 9b sind mithilfe der Dialysemaschinenkonnektoren 12a, 12b mit den Verbindungsleitungen 18a, 18b der Dialysemaschine 2 verbunden. Die Steuereinheit 14 steuert die Dialysierflüssigkeitskonzentratpumpe 16 an, um, insbesondere saures, Dialysierflüssigkeitskonzentrat aus der ersten Dialysierflüssigkeitskonzentrat-Versorgungseinrichtung 5a und/oder aus der zweiten Dialysierflüssigkeitskonzentrat-Versorgungseinrichtung 5b in eine Dialysierflüssigkeitsvorbereitungsvorrichtung 30 zu befördern. Des Weiteren ist die Dialysierflüssigkeitskonzentratpumpe 16 über eine dritte Verbindungsleitung 19 mit einem Ansaugstab 20 verbunden, über welchen, alternativ, (saures) Dialysierflüssigkeitskonzentrat aus einem externen Behälter, insbesondere in Form eines Dialysierflüssigkeitskonzentrat-Kanisters, bezogen und in die Dialysierflüssigkeitsvorbereitungsvorrichtung 30 befördert werden kann. Ferner ist die Dialysierflüssigkeitsvorbereitungsvorrichtung 30 dazu ausgebildet bzw. die Steuereinheit 14 ist dazu eingerichtet, zu ermöglichen, dass die Dialysierflüssigkeitsvorbereitungsvorrichtung 30 basisches Dialysierflüssigkeitskonzentrat über eine zweite Dialysierflüssigkeitskonzentratpumpe 22 und die Leitung 24 sowie Osmosewasser aus einem Osmosewassertank 26 über die Osmosewasserleitung 28 bezieht und mit dem sauren Dialysierflüssigkeitskonzentrat aus den Dialysierflüssigkeitskonzentrat-Versorgungseinrichtungen 5a, 5b oder aus dem Dialyseflüssigkeitskonzentrat-Kanister vermischt, um somit eine Dialysierflüssigkeit für eine nachfolgende Dialysetherapie vor- bzw. aufzubereiten. Diese Dialysierflüssigkeit wird dann über eine Abführleitung 32 hin zu einem (nicht dargestellten) Dialysator für eine extrakorporale Blutbehandlung abgeführt. Es sei an dieser Stelle darauf hingewiesen, dass die Dialyseeinheit 1 bevorzugt mehrere Dialysemaschinen aufweist. Beispielshaft ist eine weitere Dialysemaschine 34 in Figur 1 dargestellt, welche über Anschlussleitungen 35a, 35b mit den zentralen Dialysierflüssigkeitskonzentrat-Versorgungseinrichtungen 5a, 5b verbunden ist.

Die erste Verbindungsleitung 18a weist ein erstes Einlassventil 36a und ein zweites Einlassventil 38a auf, welche seriell hintereinander angeordnet und über die Steuereinheit 14 ansteuerbar sind. Zwischen den beiden Einlassventilen 38a, 38b ist zudem ein Drucksensor 40a angeordnet, welcher Druckinformationen innerhalb der ersten Verbindungsleitung 18a an die Steuereinheit 14 leitet. Gleichermaßen weist die zweite Verbindungsleitung 18b ein erstes Einlassventil 36b, ein zweites Einlassventil 38b und einen dazwischen angeordneten Drucksensor 40b auf. Durch das Öffnen der ersten Einlassventile 36a, 36b bei geschlossenen zweiten Einlassventilen 38a, 38b sind die Drucksensoren 40a, 40b in der Lage festzustellen, ob eine Anschlussleitung 9a oder 9b an die Dialysemaschine 1 und die Dialysierflüssigkeitskonzentrat-Versorgungseinrichtungen 5a, 5b angeschlossen ist oder nicht, insbesondere da die Dialysierflüssigkeitskonzentrat-Versorgungseinrichtungen 5a, 5b Dialysierflüssigkeitskonzentrat mit Überdruck durch die Ringleitungen 7a, 7b fördern.

Demnach ist die Steuereinheit 14 derart ausgebildet, um mit der Dialysierflüssigkeitsvorbereitungsvorrichtung 30, den zwei Dialysierflüssigkeitskonzentratpumpen 16, 22 und den ersten Einlassventilen 36a, 36b, den zweiten Einlassventilen 38a, 38b sowie den Drucksensoren 40a, 40b zu kommunizieren.

Figur 2 ist ein Flussdiagramm eines Verfahrens zum Vorbereiten einer Dialysetherapie. In einem ersten Schritt S1, welcher erst dann beginnt nachdem die Dialysemaschine 2 bzw. eine andere Dialysemaschine der Dialyseeinheit 1, etwa die Dialysemaschine 34, ohne einen daran angeschlossenen/ konnektierten Patienten eingeschaltet wurde oder nachdem ein Patient nach der Dialysetherapie von der Dialysemaschine 2 getrennt worden ist, wird der Hydraulikkreislauf der Dialysemaschine 2 gespült und gegebenenfalls mithilfe eines Desinfektionsmittels desinfiziert. Anschließend gelangt die Dialysemaschine 2 in einen Ruhezustand. In diesem Schritt S2 wartet die Dialysemaschine 2 bzw. die Steuereinheit 14 der Dialysemaschine 2 auf eine Bedieneranforderung /-eingabe mit Informationen für eine nachfolgende Dialysetherapie und erfasst diese. Je nach Bedarf wird im nachfolgenden Schritt S3 das für die nachfolgende Therapie nicht zu verwendende Dialysierflüssigkeitskonzentrat aus der jeweiligen Dialysierflüssigkeitskonzentrat-Versorgungseinrichtung 5a, 5b bezogen und in die Dialysierflüssigkeitsvorbereitungsvorrichtung 30 eingebracht, in welcher das Dialysierflüssigkeitskonzentrat für eine Mischung einer Dialysierflüssigkeit / Dialysierlösung verwendet wird, wodurch die entsprechenden Anschlussleitungen 9a, 9b und die Verbindungsleitungen 18a, 18b mithilfe des Dialysierflüssigkeitskonzentrats durchgespült werden. Im anschließenden Schritt S4 wird das Dialysierflüssigkeitskonzentrat, welches für die nachfolgende Therapie verwendet wird, aus der jeweiligen Dialysierflüssigkeitskonzentrat-Versorgungseinrichtung 5a, 5b bezogen und in die Dialysierflüssigkeitsvorbereitungsvorrichtung 30 eingebracht und für die Bildung der Dialysierflüssigkeit vermischt. Vorzugsweise können im Schritt S3 auch die Dialysierflüssigkeitskonzentrate beider Dialysierflüssigkeitskonzentrat-Versorgungseinrichtungen 5a, 5b nacheinander bezogen werden, damit beide Anschlussleitungen 9a, 9b und beide Verbindungsleitungen 18a, 18b durchspült werden, für den Fall, dass für die nachfolgende Therapie ein Dialysierflüssigkeitskonzentrat aus einem externen Kanister verwendet wird.

Ein Teil des Schritts S3 kann es sein, ein Spülvolumen des in der nachfolgenden Dialysetherapie nicht zu verwendenden Dialysierflüssigkeitskonzentrats in Abhängigkeit eines Schlauchdurchmessers und einer Schlauchlänge einer Anschlussleitung zu bestimmen bzw. zu berechnen bzw. abzurufen, so dass in Schritt S3 lediglich das bestimmte bzw. berechnete Spülvolumen eingebracht wird. Ferner kann ein Teil des Schritts S3 sein, festzustellen, insbesondere über einen der Drucksensoren 40a, 40b, ob die zentralen Dialysierflüssigkeitskonzentrat-Versorgungseinrichtungen 5a, 5b an die Dialysemaschine 2 angeschlossen bzw. konnektiert sind, so dass das Einbringen gemäß Schritt S3 nur bei angeschlossenen bzw. konnektierten zentralen Dialysierflüssigkeitskonzentrat-Versorgungseinrichtungen 5a, 5b erfolgt. Außerdem kann ein Teil des Schritts S3 sein, eine Inaktivität bzw. einen Stillstand der Anschlussleitungen 9a, 9b zu überwachen und das Einbringen gemäß Schritt S3 nur durchzuführen, wenn eine vorbestimmte Zeitdauer der Inaktivität verstrichen ist. Alternativ kann das Einbringen gemäß Schritt S3 auch grundsätzlich bei jedem Vorbereiten der Dialysierflüssigkeit durchgeführt werden.

Erst nachdem die vorstehend beschriebenen (Dialysierflüssigkeits-) Vorbereitungsschritte durchlaufen wurden, wird ein Patient für die nachfolgende Dialysetherapie an die Dialysemaschine 2 angeschlossen/ konnektiert und die eigentliche Dialysetherapie wird gestartet, welche nicht Teil des offenbarungsgemäßen Verfahrens ist. Nach der Dialysetherapie wird der Patient wieder von der Dialysemaschine 2 getrennt und das offenbarungsgemäße Vorbereitungsverfahren wird wieder von vorne mit Schritt S1 des Spülens/ Desinfizierens gestartet. Das Verfahren ist auf einem Computerprogramm in der Steuereinheit 14 gespeichert.

### Bezugszeichenliste

- 1: Dialyseeinheit
- 2: Dialysemaschine
- 5a, 5b: erste und zweite zentrale Dialysierflüssigkeitskonzentrat-Versorgungseinrichtung
- 6a, 6b: erstes und zweites Dialysierflüssigkeitskonzentrat-Reservoir
- 7a, 7b: erste und zweite Ringleitung
- 9a, 9b: erste und zweite Anschlussleitung
- 10a, 10b: erste und zweite Ringleitungskonnektoren
- 12a, 12b: erste und zweite Dialysemaschinenkonnektoren
- 14: Steuereinheit
- 16: erste Dialysierflüssigkeitskonzentratpumpe
- 18a, 18b: erste und zweite Verbindungsleitung
- 19: dritte Verbindungsleitung
- 22: zweite Dialysierflüssigkeitskonzentratpumpe
- 24: Leitung
- 26: Osmosetank
- 28: Osmosewasserleitung
- 30: Dialysierflüssigkeitsvorbereitungsvorrichtung
- 32: Abführleitung
- 34: (weitere) Dialysemaschine
- 35a, 35b: (zusätzliche) Anschlussleitungen
- 36a, 36b: erste Einlassventile
- 38a, 38b: zweite Einlassventile
- 40a, 40b: Drucksensoren

- S1: Schritt Spülen/ Desinfizieren
- S2: Schritt Warten und Erfassen von Bedieneranforderungen
- S3: Schritt Einbringen eines in der nachfolgenden Dialysetherapie nicht zu verwendenden Dialysierflüssigkeitskonzentrats
- S4: Schritt Einbringen eines in der nachfolgenden Dialysetherapie zu verwendenden Dialysierflüssigkeitskonzentrats

## Patentansprüche

1. Dialyseeinheit (1) mit:
einer Dialysemaschine (2), welche eine Dialysierflüssigkeitsvorbereitungsvorrichtung (30) aufweist, die vorgesehen und eingerichtet ist, um eine Dialysierflüssigkeit für eine nachfolgende Dialysetherapie vorzubereiten,
einer ersten zentralen Dialysierflüssigkeitskonzentrat-Versorgungseinrichtung (5a), welche mit der Dialysemaschine (2) über eine erste Anschlussleitung (9a) verbunden ist,
zumindest einer zweiten zentralen Dialysierflüssigkeitskonzentrat-Versorgungseinrichtung (5b), welche mit der Dialysemaschine (2) über eine zweite Anschlussleitung (9b) verbunden ist, wobei die ersten und zweiten zentralen Dialysierflüssigkeitskonzentrat-Versorgungseinrichtungen (5a, 5b) unterschiedliche Dialysierflüssigkeitskonzentrate enthalten und der Dialysierflüssigkeitsvorbereitungsvorrichtung (30) der Dialysemaschine (2) bereitstellen können, **gekennzeichnet durch** eine Steuereinheit (14), welche eingerichtet ist, beim Vorbereiten der Dialysierflüssigkeit für die nachfolgende Dialysetherapie ein in dieser nicht zu verwendendes Dialysierflüssigkeitskonzentrat oder in dieser nicht zu verwendende Dialysierflüssigkeitskonzentrate aus der ersten zentralen Dialysierflüssigkeitskonzentrat-Versorgungseinrichtung (5a) und/oder der zweiten zentralen Dialysierflüssigkeitskonzentrat-Versorgungseinrichtung (5b) in die Dialysierflüssigkeitsvorbereitungsvorrichtung (30) einzubringen und für eine Mischung der Dialysierflüssigkeit zu verwenden, um die erste Anschlussleitung (9a) und/oder die zweite Anschlussleitung (9b) zu spülen, und erst im Anschluss ein in der nachfolgenden Therapie zu verwendendes Dialysierflüssigkeitskonzentrat in die Dialysierflüssigkeitsvorbereitungsvorrichtung (30) einzubringen und für die Mischung der Dialysierflüssigkeit zu verwenden.

2. Dialyseeinheit (1) nach Anspruch 1, **dadurch gekennzeichnet, dass** die Steuereinheit (14) eingerichtet ist, eine Inaktivität der ersten und zweiten Anschlussleitungen (9a, 9b) zeitlich zu überwachen und die erste und/oder die zweite Anschlussleitung (9a, 9b) erst nach Erreichen einer vorbestimmten Zeitdauer der Inaktivität beim Vorbereiten der Dialysierflüssigkeit für die nachfolgende Dialysetherapie zu spülen, oder dass die Steuereinheit (14) eingerichtet ist, bei jedem Vorbereiten der Dialysierflüssigkeit die erste und/oder die zweite Anschlussleitung (9a, 9b) zu spülen.

3. Dialyseeinheit (1) nach Anspruch 1 oder 2, **dadurch gekennzeichnet, dass** die zentralen Dialysierflüssigkeitskonzentrat-Versorgungseinrichtungen (5a, 5b) jeweils ein Dialysierflüssigkeitskonzentrat-Reservoir (6a, 6b) zum Aufbewahren der Dialysierflüssigkeitskonzentrate und eine daran angeschlossene Ringleitung (7a, 7b) aufweisen.

4. Dialyseeinheit (1) nach Anspruch 3, **dadurch gekennzeichnet, dass** die Anschlussleitungen (9a, 9b) über Ringleitungskonnektoren (10a, 10b) mit den Ringleitungen (7a, 7b) und über Dialysemaschinenkonnektoren (12a, 12b) mit der Dialysemaschine (2) verbunden sind.

5. Dialyseeinheit (1) nach einem der vorhergehenden Ansprüche, **dadurch gekennzeichnet, dass** die erste Anschlussleitung (9a) mit einer ersten Verbindungsleitung (18a) und die zweite Anschlussleitung (9b) mit einer zweiten Verbindungsleitung (18b) in Verbindung stehen, wobei die Verbindungsleitungen (18a, 18b) innerhalb der Dialysemaschine (2) angeordnet sind, und die Dialysierflüssigkeitskonzentrate aus den zentralen Dialysierflüssigkeitskonzentrat-Versorgungseinrichtungen (5a, 5b) über die Anschlussleitungen (9a, 9b) und die Verbindungsleitungen (18a, 18b) der Dialysierflüssigkeitsvorbereitungsvorrichtung (30) zuführbar sind.

6. Dialyseeinheit (1) nach Anspruch 5, **dadurch gekennzeichnet, dass** die Verbindungsleitungen (18a, 18b) jeweils ein erstes Einlassventil (36a; 36b), ein zweites Einlassventil (38a, 38b) und einen Drucksensor (40a, 40b) aufweisen und die Steuereinheit (14) der Dialysemaschine (2) eingerichtet ist, über die Drucksensoren (40a, 40b) einen Druck in den ersten und zweiten Verbindungsleitungen (18a, 18b) zu erfassen und dadurch zu bestimmen, ob die jeweiligen Anschlussleitungen (9a, 9b) an die Dialysemaschine (2) und die zentralen Dialysierflüssigkeitskonzentrat-Versorgungseinrichtungen (5a, 5b) angeschlossen sind.

7. Dialyseeinheit (1) nach einem der vorhergehenden Ansprüche, **dadurch gekennzeichnet, dass** die Steuereinheit (14) eingerichtet ist, die erste und/oder die zweite Anschlussleitung (9a, 9b) mit einem minimal zum Spülen der Anschlussleitungen (9a, 9b) erforderlichen, insbesondere im Voraus in der Dialysemaschine (1) hinterlegten, Spülvolumen der Dialysierflüssigkeitskonzentrate zu spülen, welches dem Volumen der ersten und/oder der zweiten Anschlussleitung (9a, 9b) entspricht.

8. Dialyseeinheit (1) nach Anspruch 7, **dadurch gekennzeichnet, dass** die Steuereinheit (14) eingerichtet ist, das minimal erforderliche Spülvolumen der Dialysierflüssigkeitskonzentrate zum Spülen der Anschlussleitungen (9a, 9b) aus einem Schlauchdurchmesser und einer Schlauchlänge der jeweiligen Anschlussleitungen (9a, 9b) zu bestimmen bzw. zu berechnen, und das bestimmte bzw. berechnete Spülvolumen insbesondere über eine vorgesehene Dialysierflüssigkeitskonzentratpumpe (16) in die Dialysierflüssigkeitsvorbereitungsvorrichtung (30) einzubringen bzw. anzusaugen.

9. Dialyseeinheit (1) nach einem der vorhergehenden Ansprüche, **dadurch gekennzeichnet, dass** die Dialyseeinheit (1) zumindest eine weitere Dialysemaschine (34) aufweist, welche mit den Dialysierflüssigkeitskonzentrat-Versorgungseinrichtungen (5a, 5b) über eine erste und eine zweite zusätzliche Anschlussleitung (35a, 35b) verbunden ist, wobei die Dialysemaschine (34) eingerichtet ist, beim Vorbereiten der Dialysierflüssigkeit für die nachfolgende Dialysetherapie die erste und/oder die zweite zusätzliche Anschlussleitung (35a, 35b) durchzuspülen.

10. Dialyseeinheit (1) nach einem der vorhergehenden Ansprüche, **dadurch gekennzeichnet, dass** die Dialysemaschine (2) eine Dialysierflüssigkeitskonzentratpumpe (16) aufweist, welche ausgebildet ist, die Dialysierflüssigkeitskonzentrate aus den ersten und/oder zweiten zentralen Dialysierflüssigkeitskonzentrat-Versorgungseinrichtungen (5a, 5b) in die Dialysierflüssigkeitsvorbereitungsvorrichtung (30) zu befördern.

11. Dialyseeinheit (1) nach Anspruch 10, **dadurch gekennzeichnet, dass** die Steuereinheit (14) eingerichtet ist, das in der nachfolgenden Therapie zu verwendende Dialysierflüssigkeitskonzentrat durch die Dialysierflüssigkeitskonzentratpumpe (16) über einen Ansaugstab (20) aus einem Dialysierflüssigkeitskonzentrat-Kanister anzusaugen und für die Mischung der Dialysierflüssigkeit in der Dialysierflüssigkeitsvorbereitungsvorrichtung (30) zu verwenden.

12. Dialyseeinheit (1) nach einem der vorhergehenden Ansprüche, **dadurch gekennzeichnet, dass** die Dialysierflüssigkeitsvorbereitungsvorrichtung (30) ausgebildet ist, die Dialysierflüssigkeit für die nachfolgende Dialysetherapie aus einem sauren Dialysierflüssigkeitskonzentrat aus einer der zentralen Dialysierflüssigkeitskonzentrat-Versorgungseinrichtungen (5a, 5b) oder eines externen Kanisters, aus Osmosewasser aus einem Osmosetank (26) oder einer zentralen RO-Anlage/Versorgungsleitung und aus einem weiteren basischen Dialysierflüssigkeitskonzentrat vorzubereiten.

13. Verfahren zum Vorbereiten einer Dialysetherapie, insbesondere zum Vorbereiten einer Dialysierflüssigkeit für eine nachfolgende Dialysetherapie, welches beginnt, nachdem eine Dialysemaschine (2) ohne einen daran angeschlossenen Patienten eingeschaltet wurde oder nachdem ein Patient von der Dialysemaschine (2) getrennt wurde, und welches endet, bevor ein Patient an die Dialysemaschine (2) angeschlossen wird, mit den Schritten:
Einbringen (S3) eines in der nachfolgenden Dialysetherapie nicht zu verwendenden Dialysierflüssigkeitskonzentrats oder von in der nachfolgenden Dialysetherapie nicht zu verwendenden Dialysierflüssigkeitskonzentraten aus einer ersten zentralen Dialysierflüssigkeitskonzentrat-Versorgungseinrichtung (5a) und/oder einer zweiten zentralen Dialysierflüssigkeitskonzentrat-Versorgungseinrichtung (5b) in eine Dialysierflüssigkeitsvorbereitungsvorrichtung (30) der Dialysemaschine (2) und Verwenden des in der nachfolgenden Dialysetherapie nicht zu verwendenden Dialysierflüssigkeitskonzentrats oder der in der nachfolgenden Dialysetherapie nicht zu verwendenden Dialysierflüssigkeitskonzentrate für eine Mischung der Dialysierflüssigkeit; und im Anschluss,
Einbringen (S4) eines in der nachfolgenden Dialysetherapie zu verwendenden Dialysierflüssigkeitskonzentrats in die Dialysierflüssigkeitsvorbereitungsvorrichtung (30) der Dialysemaschine (2) und Verwenden des in der nachfolgenden Dialysetherapie zu verwendenden Dialysierflüssigkeitskonzentrats für die Mischung der Dialysierflüssigkeit.

14. Verfahren nach Anspruch 13, **gekennzeichnet durch** Schritte:
Bestimmen oder Berechnen oder Abrufen eines minimalen Spülvolumens des in der nachfolgenden Dialysetherapie nicht zu verwendenden Dialysierflüssigkeitskonzentrats oder der in der nachfolgenden Dialysetherapie nicht zu verwendenden Dialysierflüssigkeitskonzentrate, bevorzugt in Abhängigkeit eines Schlauchdurchmessers und einer Schlauchlänge einer Anschlussleitung (9a, 9b), welche die entsprechende zentrale Dialysierflüssigkeitskonzentrat-Versorgungseinrichtungen (5a, 5b) mit der Dialysemaschine (2) verbindet; und
Einbringen des bestimmten oder berechneten oder abgerufenen minimalen Spülvolumens, insbesondere über eine vorgesehene Dialysierflüssigkeitskonzentratpumpe (16).

15. Verfahren nach Anspruch 13 oder 14, **gekennzeichnet durch** den zusätzlichen Schritt:
Feststellen, insbesondere über einen Drucksensor (40a, 40b), ob eine oder mehrere zentrale Dialysierflüssigkeitskonzentrat-Versorgungseinrichtungen (5a, 5b) an die Dialysemaschine (2) angeschlossen bzw. konnektiert sind.

16. Computerprogramm, umfassend Befehle, die bei einer Ausführung durch einen Computer diesen veranlassen, die folgenden Verfahrensschritte, insbesondere gemäß dem Verfahren nach einem der Ansprüche 13 bis 15, auszuführen:
Einbringen (S3) eines in einer nachfolgenden Dialysetherapie nicht zu verwendenden Dialysierflüssigkeitskonzentrats oder von in der nachfolgenden Dialysetherapie nicht zu verwendenden Dialysierflüssigkeitskonzentraten aus einer ersten zentralen Dialysierflüssigkeitskonzentrat-Versorgungseinrichtung (5a) und/oder einer zweiten zentralen Dialysierflüssigkeitskonzentrat-Versorgungseinrichtung (5b) in eine Dialysierflüssigkeitsvorbereitungsvorrichtung (30) einer Dialysemaschine (2) und Verwenden des in der nachfolgenden Dialysetherapie nicht zu verwendenden Dialysierflüssigkeitskonzentrat oder der in der nachfolgenden Dialysetherapie nicht zu verwendenden Dialysierflüssigkeitskonzentrate für eine Mischung der Dialysierflüssigkeit; und im Anschluss,
Einbringen (S4) eines in der nachfolgenden Dialysetherapie zu verwendenden Dialysierflüssigkeitskonzentrat in die Dialysierflüssigkeitsvorbereitungsvorrichtung (30) der Dialysemaschine (2) und Verwenden des in der nachfolgenden Dialysetherapie zu verwendenden Dialysierflüssigkeitskonzentrats für die Mischung der Dialysierflüssigkeit.

## Claims

1. Dialysis unit (1) comprising:
a dialysis machine (2) having a dialysis fluid preparation device (30) provided and configured to prepare a dialysis fluid for subsequent dialysis therapy,
a first central dialysis fluid concentrate supply device (5a), which is connected to the dialysis machine (2) via a first connecting line (9a),
at least one second central dialysis fluid concentrate supply device (5b), which is connected to the dialysis machine (2) via a second connecting line (9b), wherein the first and second central dialysis fluid concentrate supply devices (5a, 5b) contain different dialysis fluid concentrates and can supply them to the dialysis fluid preparation device (30) of the dialysis machine (2), **characterized by** a control unit (14) which is configured, when preparing the dialysis fluid for the subsequent dialysis therapy, to introduce a dialysis fluid concentrate or dialysis fluid concentrates not to be used in the dialysis fluid from the first central dialysis fluid concentrate supply device (5a) and/or the second central dialysis fluid concentrate supply device (5b) into the dialysis fluid preparation device (30) and to use it for mixing the dialysis fluid in order to flush the first connecting line (9a) and/or the second connecting line (9b), and only then introduce a dialysis fluid concentrate to be used in the subsequent therapy into the dialysis fluid preparation device (30) and use it for mixing the dialysis fluid.

2. The dialysis unit (1) according to claim 1, **characterized in that** the control unit (14) is configured to monitor an inactivity of the first and second connecting lines (9a, 9b) over time and to flush the first and/or second connecting line(s) (9a, 9b) only after a predetermined period of inactivity has been reached when preparing the dialysis fluid for the subsequent dialysis therapy, or that the control unit (14) is configured to flush the first and/or second connecting line(s) (9a, 9b) each time the dialysis fluid is prepared.

3. The dialysis unit (1) according to claim 1 or 2, **characterized in that** the central dialysis fluid concentrate supply devices (5a, 5b) each have a dialysis fluid concentrate reservoir (6a, 6b) for storing the dialysis fluid concentrates and a ring line (7a, 7b) connected thereto.

4. The dialysis unit (1) according to claim 3, **characterized in that** the connecting lines (9a, 9b) are connected to the ring lines (7a, 7b) via ring line connectors (10a, 10b) and to the dialysis machine (2) via dialysis machine connectors (12a, 12b).

5. The dialysis unit (1) according to any of the preceding claims, **characterized in that** the first connecting line (9a) is connected to a first interconnect line (18a) and the second connecting line (9b) is connected to a second interconnect line (18b), wherein the interconnect lines (18a, 18b) are arranged inside the dialysis machine (2), and the dialysis fluid concentrates can be supplied from the central dialysis fluid concentrate supply devices (5a, 5b) via the connecting lines (9a, 9b) and the interconnect lines (18a, 18b) of the dialysis fluid preparation device (30).

6. The dialysis unit (1) according to claim 5, **characterized in that** the interconnect lines (18a, 18b) each have a first inlet valve (36a; 36b), a second inlet valve (38a, 38b), and a pressure sensor (40a, 40b), and the control unit (14) of the dialysis machine (2) is configured to detect a pressure in the first and second interconnect lines (18a, 18b) via the pressure sensors (40a, 40b) and thereby determine whether the respective connecting lines (9a, 9b) are connected to the dialysis machine (2) and the central dialysis fluid concentrate supply devices (5a, 5b).

7. The dialysis unit (1) according to any of the preceding claims, **characterized in that** the control unit (14) is configured to flush the first and/or second connecting line (9a, 9b) with a minimum flush volume of the dialysis fluid concentrates stored in advance in the dialysis machine (1) and required for flushing the connecting lines (9a, 9b), which corresponds to the volume of the first and/or second connecting line (9a, 9b).

8. The dialysis unit (1) according to claim 7, **characterized in that** the control unit (14) is configured to determine or calculate the flush volume of the dialysis fluid concentrates required at the minimum for flushing the connecting lines (9a, 9b) from a tube diameter and a tube length of the respective connecting lines (9a, 9b), and to introduce or suck the determined or calculated flush volume into the dialysis fluid preparation device (30), in particular via a provided dialysis fluid concentrate pump (16).

9. The dialysis unit (1) according to any of the preceding claims, **characterized in that** the dialysis unit (1) has at least one further dialysis machine (34) which is connected to the dialysis fluid concentrate supply devices (5a, 5b) via a first and a second additional connecting line (35a, 35b), wherein the dialysis machine (34) is configured to flush the first and/or second additional connecting line (35a, 35b) when preparing the dialysis fluid for subsequent dialysis therapy.

10. The dialysis unit (1) according to any of the preceding claims, **characterized in that** the dialysis machine (2) has a dialysis fluid concentrate pump (16) which is configured to convey the dialysis fluid concentrates from the first and/or second central dialysis fluid concentrate supply devices (5a, 5b) into the dialysis fluid preparation device (30).

11. The dialysis unit (1) according to claim 10, **characterized in that** the control unit (14) is designed to draw the dialysis fluid concentrate to be used in the subsequent therapy through the dialysis fluid concentrate pump (16) via a suction rod (20) from a dialysis fluid concentrate canister and to use it for mixing the dialysis fluid in the dialysis fluid preparation device (30).

12. The dialysis unit (1) according to any of the preceding claims, **characterized in that** the dialysis fluid preparation device (30) is configured to prepare the dialysis fluid for subsequent dialysis therapy from an acidic dialysis fluid concentrate from one of the central dialysis fluid concentrate supply devices (5a, 5b) or an external canister, from osmosis water from an osmosis tank (26) or a central RO plant/supply line, and from a further alkaline dialysis fluid concentrate.

13. A method for preparing dialysis therapy, in particular for preparing a dialysis fluid for subsequent dialysis therapy, which begins after a dialysis machine (2) was switched on without a patient connected to it or after a patient was disconnected from the dialysis machine (2), and which ends before a patient is connected to the dialysis machine (2), the method comprising the steps of:
introducing (S3) a dialysis fluid concentrate or dialysis fluid concentrates not to be used in the subsequent dialysis therapy from a first central dialysis fluid concentrate supply device (5a) and/or a second central dialysis fluid concentrate supply device (5b) into a dialysis fluid preparation device (30) of the dialysis machine (2) and using the dialysis fluid concentrate or dialysis fluid concentrates not to be used in the subsequent dialysis therapy for mixing the dialysis fluid; and subsequently,
introducing (S4) a dialysis fluid concentrate to be used in the subsequent dialysis therapy into the dialysis fluid preparation device (30) of the dialysis machine (2) and using the dialysis fluid concentrate to be used in the subsequent dialysis therapy for mixing the dialysis fluid.

14. The method according to claim 13, **characterized by** the steps:
determining or calculating or retrieving a minimum flush volume of the dialysis fluid concentrate or concentrates not to be used in the subsequent dialysis therapy, preferably based on a tube diameter and a tube length of a connecting line (9a, 9b) which connects the corresponding central dialysis fluid concentrate supply devices (5a, 5b) to the dialysis machine (2); and
introducing the determined or calculated or retrieved minimum flush volume, in particular via a provided dialysis fluid concentrate pump (16).

15. The method according to claim 13 or 14, **characterized by** the additional step of:
determining, in particular via a pressure sensor (40a, 40b), whether one or more central dialysis fluid concentrate supply devices (5a, 5b) are connected to the dialysis machine (2).

16. A computer program comprising instructions which, when executed by a computer, cause the computer to perform the following method steps, in particular according to the method according to any of claims 13 to 15:
introducing (S3) a dialysis fluid concentrate or dialysis fluid concentrates not to be used in subsequent dialysis therapy from a first central dialysis fluid concentrate supply device (5a) and/or a second central dialysis fluid concentrate supply device (5b) into the dialysis fluid preparation device (30) of a dialysis machine (2) and using the dialysis fluid concentrate or dialysis fluid concentrates not to be used in subsequent dialysis therapy for mixing the dialysis fluid; and subsequently,
introducing (S4) a dialysis fluid concentrate to be used in subsequent dialysis therapy into the dialysis fluid preparation device (30) of the dialysis machine (2) and using the dialysis fluid concentrate to be used in the subsequent dialysis therapy for mixing the dialysis fluid.

## Revendications

1. Unité de dialyse (1) avec :
une machine de dialyse (2) qui présente un dispositif de préparation de liquide de dialyse (30) qui est prévu et conçu afin de préparer un liquide de dialyse pour une thérapie de dialyse suivante,
un premier appareil d'alimentation en concentré de liquide de dialyse (5a) central qui est relié à la machine de dialyse (2) par le biais d'une première conduite de raccordement (9a),
au moins un second appareil d'alimentation en concentré de liquide de dialyse (5b) central qui est relié à la machine de dialyse (2) par le biais d'une seconde conduite de raccordement (9b), dans laquelle les premier et second appareils d'alimentation en concentré de liquide de dialyse (5a, 5b) centraux contiennent différents concentrés de liquide de dialyse et peuvent les fournir au dispositif de préparation de liquide de dialyse (30) de la machine de dialyse (2), **caractérisée par** un dispositif de commande (14) qui est conçu afin d'introduire lors de la préparation du liquide de dialyse pour la thérapie de dialyse suivante un concentré de liquide de dialyse à ne pas utiliser dans celle-ci ou des concentrés de liquide de dialyse à ne pas utiliser dans celle-ci provenant du premier appareil d'alimentation en concentré de liquide de dialyse (5a) central et/ou du second appareil d'alimentation en concentré de liquide de dialyse (5b) central dans le dispositif de préparation de liquide de dialyse (30) et de l'utiliser ou les utiliser pour un mélange du liquide de dialyse afin de rincer la première conduite de raccordement (9a) et/ou la seconde conduite de raccordement (9b), et d'introduire seulement ensuite un concentré de liquide de dialyse à utiliser dans la thérapie suivante dans le dispositif de préparation de liquide de dialyse (30) et de l'utiliser pour le mélange du liquide de dialyse.

2. Unité de dialyse (1) selon la revendication 1, **caractérisée en ce que** le dispositif de commande (14) est conçu afin de surveiller temporellement une inactivité des première et seconde conduites de raccordement (9a, 9b) et de rincer la première et/ou la seconde conduite de raccordement (9a, 9b) seulement après qu'une durée prédéterminée de l'inactivité lors de la préparation du liquide de dialyse pour la thérapie de dialyse suivante soit atteinte, ou **en ce que** le dispositif de commande (14) est conçu afin de rincer la première et/ou la seconde conduite de raccordement (9a, 9b) lors de chaque préparation du liquide de dialyse.

3. Unité de dialyse (1) selon la revendication 1 ou 2, **caractérisée en ce que** les appareils d'alimentation en concentré de liquide de dialyse (5a, 5b) centraux présentent respectivement un réservoir de concentré de liquide de dialyse (6a, 6b) pour la conservation des concentrés de liquide de dialyse et une conduite annulaire (7a, 7b) raccordée à celui-ci.

4. Unité de dialyse (1) selon la revendication 3, **caractérisée en ce que** les conduites de raccordement (9a, 9b) sont reliées aux conduites annulaires (7a, 7b) par le biais de connecteurs de conduite annulaire (10a, 10b) et à la machine de dialyse (2) par le biais de connecteurs de machine de dialyse (12a, 12b).

5. Unité de dialyse (1) selon l'une quelconque des revendications précédentes, **caractérisée en ce que** la première conduite de raccordement (9a) est en liaison avec une première conduite de liaison (18a) et la seconde conduite de raccordement (9b) avec une seconde conduite de liaison (18b), dans laquelle les conduites de liaison (18a, 18b) sont agencées à l'intérieur de la machine de dialyse (2), et les concentrés de liquide de dialyse provenant des appareils d'alimentation en concentré de liquide de dialyse (5a, 5b) centraux peuvent être amenés au dispositif de préparation de liquide de dialyse (30) par le biais des conduites de raccordement (9a, 9b) et des conduites de liaison (18a ; 18b).

6. Unité de dialyse (1) selon la revendication 5, **caractérisée en ce que** les conduites de liaison (18a, 18b) présentent respectivement une première vanne d'entrée (36a ; 36b), une seconde vanne d'entrée (38a, 38b) et un capteur de pression (40a, 40b), et le dispositif de commande (14) de la machine de dialyse (2) est conçu afin de détecter une pression dans les première et seconde conduites de liaison (18a, 18b) par le biais des capteurs de pression (40a, 40b) et de déterminer ainsi si les conduites de raccordement (9a, 9b) respectives sont raccordées à la machine de dialyse (2) et aux appareils d'alimentation en concentré de liquide de dialyse (5a, 5b) centraux.

7. Unité de dialyse (1) selon l'une quelconque des revendications précédentes, **caractérisée en ce que** le dispositif de commande (14) est conçu afin de rincer la première et/ou la seconde conduite de raccordement (9a, 9b) avec un volume de rinçage minimal nécessaire des concentrés de liquide de dialyse pour le rinçage des conduites de raccordement (9a, 9b), versé en particulier au préalable dans la machine de dialyse (1), volume qui correspond au volume de la première et/ou de la seconde conduite de raccordement (9a, 9b).

8. Unité de dialyse (1) selon la revendication 7, **caractérisée en ce que** le dispositif de commande (14) est conçu afin de déterminer ou calculer le volume de rinçage minimal nécessaire des concentrés de liquide de dialyse pour le rinçage des conduites de raccordement (9a, 9b) à partir d'un diamètre de tuyau et d'une longueur de tuyau des conduites de raccordement (9a, 9b) respectives, et d'introduire ou d'aspirer le volume de rinçage déterminé ou calculé en particulier par le biais d'une pompe de concentré de liquide de dialyse (16) prévue dans le dispositif de préparation de liquide de dialyse (30).

9. Unité de dialyse (1) selon l'une quelconque des revendications précédentes, **caractérisée en ce que** l'unité de dialyse (1) présente au moins une autre machine de dialyse (34) qui est reliée aux appareils d'alimentation en concentré de liquide de dialyse (5a, 5b) par le biais d'une première et d'une seconde conduite de raccordement supplémentaire (35a, 35b), dans laquelle la machine de dialyse (34) est conçue afin de rincer la première et/ou la seconde conduite de raccordement supplémentaire (35a, 35b) lors de la préparation du liquide de dialyse pour la thérapie de dialyse suivante.

10. Unité de dialyse (1) selon l'une quelconque des revendications précédentes, **caractérisée en ce que** la machine de dialyse (2) présente une pompe de concentré de liquide de dialyse (16) qui est configurée afin de transporter les concentrés de liquide de dialyse provenant des premier et/ou second appareils d'alimentation en concentré de liquide de dialyse (5a, 5b) centraux dans l'appareil de préparation de liquide de dialyse (30).

11. Unité de dialyse (1) selon la revendication 10, **caractérisée en ce que** le dispositif de commande (14) est conçu afin d'aspirer à partir d'un bidon de concentré de liquide de dialyse le concentré de liquide de dialyse à utiliser dans la thérapie suivante par la pompe de concentré de liquide de dialyse (16) par le biais d'une barre d'aspiration (20) et de l'utiliser pour le mélange du liquide de dialyse dans le dispositif de préparation de liquide de dialyse (30).

12. Unité de dialyse (1) selon l'une quelconque des revendications précédentes, **caractérisée en ce que** le dispositif de préparation de liquide de dialyse (30) est configurée afin de préparer le liquide de dialyse pour la thérapie de dialyse suivante à partir d'un concentré de liquide de dialyse acide provenant d'un des appareils d'alimentation en concentré de liquide de dialyse (5a, 5b) centraux ou d'un bidon externe, à partir d'eau osmosée d'un réservoir d'eau osmosée (26) ou d'une conduite d'alimentation/d'installation d'osmose centrale et à partir d'un autre concentré de liquide de dialyse basique.

13. Procédé de préparation d'une thérapie de dialyse, en particulier de préparation d'un liquide de dialyse pour une thérapie de dialyse suivante qui commence après qu'une machine de dialyse (2) a été mise en service sans patient raccordé à celle-ci ou après qu'un patient a été séparé de la machine de dialyse (2) et qui se termine avant qu'un patient ne soit raccordé à la machine de dialyse (2), avec les étapes suivantes :
l'introduction (S3) d'un concentré de liquide de dialyse à ne pas utiliser dans la thérapie de dialyse suivante ou de concentrés de liquide de dialyse à ne pas utiliser dans la thérapie de dialyse suivante provenant d'un premier appareil d'alimentation en concentré de liquide de dialyse (5a) central et/ou d'un second appareil d'alimentation en concentré de liquide de dialyse central (5b) dans un dispositif de préparation de liquide de dialyse (30) de la machine de dialyse (2) et l'utilisation du concentré de liquide de dialyse à ne pas utiliser dans la thérapie de dialyse suivante ou des concentrés de liquide de dialyse à ne pas utiliser dans la thérapie de dialyse suivante pour un mélange du liquide de dialyse ; et ensuite
l'introduction (S4) d'un concentré de liquide de dialyse à utiliser dans la thérapie de dialyse suivante dans le dispositif de préparation de liquide de dialyse (30) de la machine de dialyse (2) et l'utilisation du concentré de liquide de dialyse à utiliser dans la thérapie de dialyse suivante pour le mélange du liquide de dialyse.

14. Procédé selon la revendication 13, **caractérisé par** les étapes suivantes :
la détermination ou le calcul ou la consultation d'un volume de rinçage minimal du concentré de liquide de dialyse à ne pas utiliser dans la thérapie de dialyse suivante ou des concentrés de liquide de dialyse à ne pas utiliser dans la thérapie de dialyse suivante, de préférence en fonction d'un diamètre de tuyau et d'une longueur de tuyau d'une conduite de raccordement (9a, 9b) qui relie les appareils d'alimentation en concentré de liquide de dialyse (5a, 5b) centraux correspondants à la machine de dialyse (2) ; et
l'introduction du volume de rinçage minimal déterminé ou calculé ou consulté, en particulier par le biais d'une pompe de concentré de liquide de dialyse (16) prévue.

15. Procédé selon la revendication 13 ou 14, **caractérisé par** l'étape supplémentaire :
la constatation, en particulier par le biais d'un capteur de pression (40a, 40b), si un ou plusieurs appareils d'alimentation en concentré de liquide de dialyse (5a, 5b) centraux sont raccordés ou connectés à la machine de dialyse.

16. Programme informatique comprenant des ordres qui amènent un ordinateur lors d'une exécution par celui-ci, à exécuter les étapes de procédé suivantes en particulier selon le procédé selon l'une quelconque des revendications 13 à 15 :
l'introduction (S3) d'un concentré de liquide de dialyse à ne pas utiliser dans une thérapie de dialyse suivante ou de concentrés de liquide de dialyse à ne pas utiliser dans la thérapie de dialyse suivante provenant d'un premier appareil d'alimentation en concentré de liquide de dialyse (5a) central et/ou d'un second appareil d'alimentation en concentré de liquide de dialyse central (5b) dans un dispositif de préparation de liquide de dialyse (30) d'une machine de dialyse (2) et l'utilisation du concentré de liquide de dialyse à ne pas utiliser dans la thérapie de dialyse suivante ou des concentrés de liquide de dialyse à ne pas utiliser dans la thérapie de dialyse suivante pour un mélange du liquide de dialyse ; et ensuite
l'introduction (S4) d'un concentré de liquide de dialyse à utiliser dans la thérapie de dialyse suivante dans le dispositif de préparation de liquide de dialyse (30) de la machine de dialyse (2) et l'utilisation du concentré de liquide de dialyse à utiliser dans la thérapie de dialyse suivante pour le mélange du liquide de dialyse.
